# EUROPÄISCHE PATENTSCHRIFT

(11) **EP 2 395 926 B1**
(45) Veröffentlichungstag und Bekanntmachung des Hinweises auf die Patenterteilung: **13.08.2014**
(21) Anmeldenummer: 10700861.7
(22) Anmeldetag: 15.01.2010
(51) Int. Cl.: A61B 17/04, A61B 17/072, A61B 17/64

(54) **CHIRURGISCHE KLAMMEREINRICHTUNG**
SURGICAL CLAMP DEVICE
AGENCEMENT DE PINCES CHIRURGICALES

(30) Priorität: 12.02.2009 AT 2392009
(43) Veröffentlichungstag der Anmeldung: 21.12.2011
(73) Patentinhaber: Klaffenböck, Johann, 5350 Strobl (AT); Klaffenböck, Lukas, 5350 Strobl (AT)
(72) Erfinder: Klaffenböck, Johann, 5350 Strobl (AT); Klaffenböck, Lukas, 5350 Strobl (AT)
(74) Vertreter: Babeluk, Michael
(86) Internationale Anmeldenummer: PCT/EP2010/050437
(87) Internationale Veröffentlichungsnummer: WO 2010/091913

(56) Entgegenhaltungen:
- WO-A1-2008/070763
- US-A1- 2004 133 238
- US-A1- 2004 138 683
- US-A1- 2005 049 599

## Beschreibung

Die Erfindung betrifft eine chirurgische Klammereinrichtung mit einem ersten und einem zweiten Plattenelement, sowie zumindest zwei Stiften, über die das erste Plattenelement mit dem zweiten Plattenelement verbindbar ist, und die zumindest zwei Stifte an einem Ende über zumindest einen Faden miteinander in Verbindung stehen, der die Stifte an dem ersten Plattenelement fixiert dadurch gekennzeichnet, daß je Stift ein Schaft vorgesehen ist, der an einem Ende den Stift trägt, während das andere Ende mit dem ersten Plattenelement in Verbindung steht.

Klammereinrichtungen werden in der Chirurgie insbesondere zur Verbindung von Gewebeteilen eingesetzt. So beschreibt die US 2004/0059349 A1 und US 2004/133238 eine derartige Klammereinrichtung, wobei zwei Stifte in einem Plattenelement untergebracht sind, die beim chirurgischen Einsatz aus der Platte herausgeklappt und in eine zweite Platte eingerastet werden, wobei zwischen den beiden Platten das zu vernähende Gewebe angeordnet ist. Nachteilig an dieser Vorrichtung ist es, dass die Klammereinrichtung aufgrund ihres komplexen Aufbaus teuer in der Fertigung ist. Des weiteren gibt es Anwendungen in der chirurgischen Medizin, bei welchen die Klammereinrichtung beispielsweise nach Verheilen des Gewebes wieder entnommen werden muss. Diese Klammereinrichtung lässt sich jedoch nur sehr aufwendig entfernen, wobei es während der Entfernung der Klammer wiederum zu Verletzungen des Gewebes kommen kann. Ähnliche Einrichtung sind auch der US 2004/138683 A1 oder der WO 2008/070763 a1 zu entnehmen.

Die EP 0 592 000 B1 beschreibt ebenfalls eine chirurgische Klammervorrichtung, mit deren Hilfe eine klammerartige Naht zum Verschließen von Wunden aufgebracht wird. Die EP 1 330 189 B1 beschreibt klammerartige Einrichtungen zur Reparatur von Mitralklappen, die auf Plikationsbändern aufgebracht sind. Auch diese Vorrichtungen verursachen bei ihrem Entfernen aus dem Gewebe Verletzungen.

In der US 2004/133238 A1 ist eine Klammereinrichtung der eingangs erwähnten Art beschrieben, wobei auch diese Klammer beim Entfernen Verletzungen im Gewebe verursacht.

Es ist daher Aufgabe der Erfindung, die oben stehenden Nachteile des Standes der Technik zu beseitigen, und eine Klammereinrichtung zur Verfügung zu stellen, die kostengünstig herstellbar und ohne wesentliche Verletzungen des Gewebes aus demselben entfernbar ist.

Diese Aufgabe wird erfindungsgemäß durch eine chirurgische Klammereinrichtung der eingangs erwähnten Art dadurch gelöst, dass je Stift ein Schaft vorgesehen ist, der an einem Ende den Stift trägt, während das andere Ende mit dem ersten Plattenelement in Verbindung steht.

Durch die Fixierung der zumindest zwei Stifte an dem ersten Plattenelement werden die Stifte parallel zueinander angeordnet, während gleichzeitig auch die Plattenelemente parallel zueinander angeordnet sind, wobei sich das zu klammernde Gewebe zwischen erstem und zweitem Plattenelement befindet. Wird nun der zumindest eine Faden durchtrennt, wird diese geometrische Anordnung aufgelöst, so dass sich die chirurgische Klammereinrichtung leicht aus dem Gewebe entfernen lässt.

Die Entfernbarkeit der Klammereinrichtung wird erfindungsgemäß dadurch verbessert, dass die zumindest zwei Stifte über eine zusätzliche lösbare Verbindung an dem ersten Plattenelement angeordnet sind, die bei Durchtrennung des zumindest einen Fadens gelöst ist. Bei Durchtrennung des Fadens zerfällt somit die erfindungsgemäße Klammereinrichtung in zwei Teile, die einfach aus dem Gewebe gezogen werden können.

Hierbei ist diese lösbare Verbindung dadurch realisiert, dass je Stift ein Schaft vorgesehen ist, der an einem Ende den Stift trägt, während das andere Ende mit dem ersten Plattenelement in Verbindung steht. Bei Durchtrennung des zumindest einen Fadens wird der Stift vom Schaft getrennt, so dass die Klammereinrichtung wiederum in zwei Teile auseinander fällt, die ohne Widerstand aus dem Gewebe gezogen werden können.

Die Anordnung des Schafts an dem ersten Plattenelement kann auf unterschiedliche Weise erfolgen. In einer ersten Ausführung ist der Schaft in einer normal auf die Plattenebene stehende Bohrung des ersten Plattenelements einsetzbar. Hierbei weist der Schaft an seinem den ersten Plattenelement zugewandten Ende vorteilhafterweise einen Kopf auf, dessen Durchmesser größer ist als der Durchmesser der Bohrung. Damit ist gewährleistet, dass der Schaft in der Bohrung positionsstabil fixiert ist. Des Weiteren hat diese Ausführung den Vorteil, dass der Faden beabstandet von dem ersten Plattenelement angeordnet ist, wobei er gleichzeitig parallel zu deren Oberfläche verläuft, sodass bei Durchtrennung des Fadens das Schneidwerkzeug in diesen Abstand zumindest teilweise eingeführt werden kann, was das Entzweischneiden des Fadens wesentlich erleichtert.

In einer anderen Variante ist der Schaft einstückig mit dem ersten Plattenelement gefertigt. Durch diese Maßnahme verringert sich die Zahl der Einzelteile der erfindungsgemäßen Klammereinrichtung, wodurch diese einfacher handhabbar wird.

Um ein stabiles Zusammensetzen der erfindungsgemäßen Klammereinrichtung zu erzielen, weist das zweite Plattenelement zumindest zwei normal auf die Plattenebene stehende Bohrungen auf, in die die zumindest zwei Stifte einsetzbar sind. Alternativ hierzu kann auch vorgesehen sein, dass die Stifte über Aufnahmen mit dem zweiten Plattenelement verbunden sind.

Die Stabilität der Anordnung der Stifte auf dem zweiten Plattenelement wird weiter dadurch erhöht, dass vorteilhafterweise das zweite Plattenelement zumindest ein Fixierelement aufweist. Vorteilhafterweise weist hierbei der Stift eine im Wesentlichen kegelförmige Spitze auf, sowie in unmittelbarer Nachbarschaft der Spitze einen reduzierten Querschnitt, wodurch eine Einschnürung gebildet wird, in die das auf dem zweiten Plattenelement angeordnete Fixierungsmittel einrastbar ist. Dabei weist das Fixierungsmittel eine im Wesentlichen der Einschnürung entsprechende Form auf, wobei in einer besonders bevorzugten und einfach realisierbaren Ausführung der Erfindung das Fixierungsmittel als zumindest eine auf der dem ersten Plattenelement abgewandten Oberfläche des ersten Plattenelements angeordnete Rastleiste ausgebildet ist.

Bei einer besonders bevorzugten Ausführung der Erfindung ist das Ende des zumindest einen Fadens unlösbar mit dem Stift verbunden. Dadurch wird gewährleistet, dass die Entfernung der erfindungsgemäßen Klammereinrichtung erst dann erfolgen kann, wenn der zumindest eine Faden durchtrennt wird.

Für den Einsatz der erfindungsgemäßen chirurgischen Klammereinrichtung muss diese aus einem Material hergestellt sein, das für den chirurgischen Einsatz geeignet ist. Besonders bevorzugt hierbei ist, dass die Plattenelemente sowie die Stifte aus Titan oder Nitinol gefertigt sind, während der zumindest eine Faden beispielsweise aus einem elastischen Material gefertigt ist.

Die erfindungsgemäße Klammereinrichtung hat sich zur Verwendung zur operativen Abheftung von Gewebe, insbesondere zur Magenplikation bewährt. Bei einer gastroösophagialen Refluxkrankheit (GERD) fließt der sauere Mageninhalt fälschlicherweise vom Magen in die Speiseröhre. Zur Behandlung dieser Erkrankung wird zur Verringerung des gastroösophagialen Reflux endoskopisch Magengewebe jenseits der gastroösophagialen Verbindung mit Hilfe von klammerartigen Elementen zusammengekniffen, um das Gewebe zu rekonfigurieren. Ähnliche Anwendungsgebiete finden sich beispielsweise auch im Darm.

Im Folgenden wird anhand von nicht-einschränkenden Ausführungsbeispielen mit zugehörigen Figuren die Erfindung näher erläutert. Darin zeigen:
- Fig. 1: eine erste perspektivische Ansicht der Klammereinrichtung;
- Fig. 2: eine zweite perspektivische Ansicht;
- Fig. 3: eine erste Seitenansicht;
- Fig. 4: eine zweite Seitenansicht;
- Fig. 5: eine Schnittansicht der Klammereinrichtung aus Fig. 1; und
- Fig. 6: bis Fig. 8 eine weitere Ausführungsvariante der Erfindung.

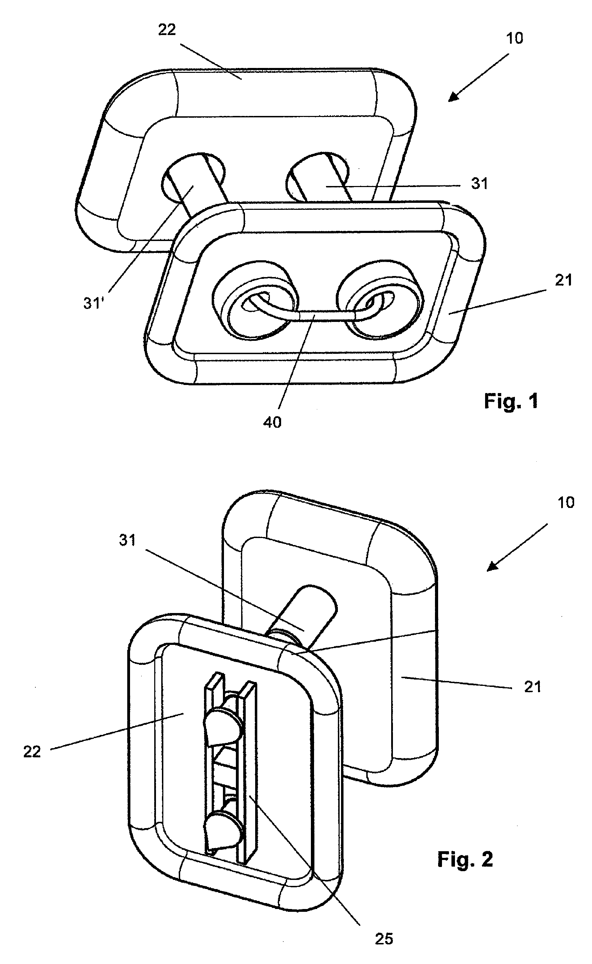

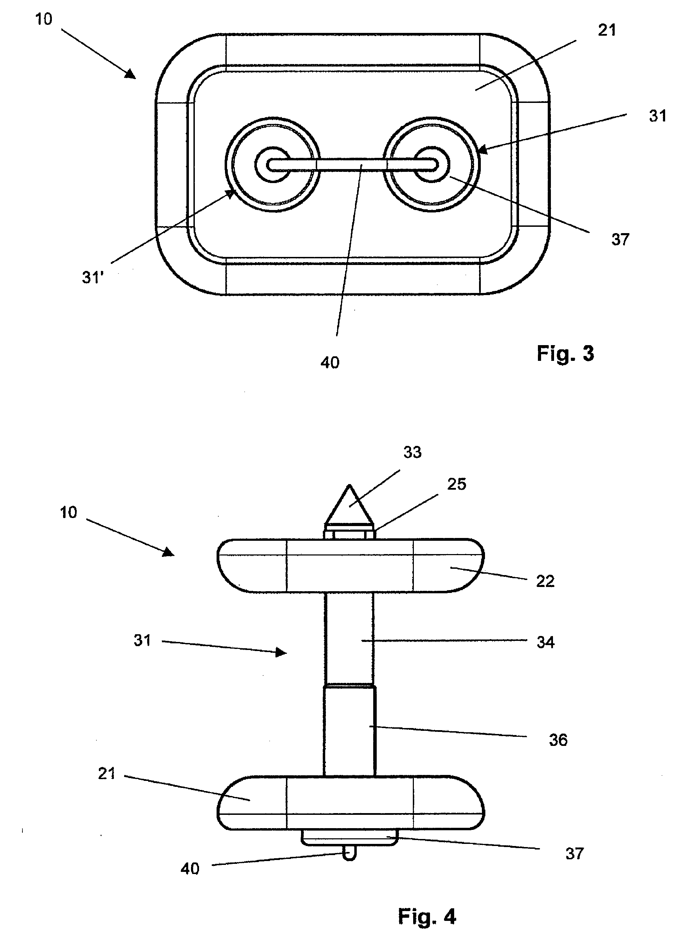

Wie in den Fig. 1 bis Fig. 4 gezeigt, weist die erfindungsgemäße Klammereinrichtung 10 zwei Plattenelemente 21, 22 auf, die über zwei Stifte 31, 31' miteinander in Verbindung stehen. Die beiden Stifte 31, 31' sind hierbei mittels eines Fadens 40 derart miteinander verbunden, dass der Faden 40 an der dem zweiten Plattenelement 22 abgewandten Oberfläche des ersten Plattenelements 21 verläuft.

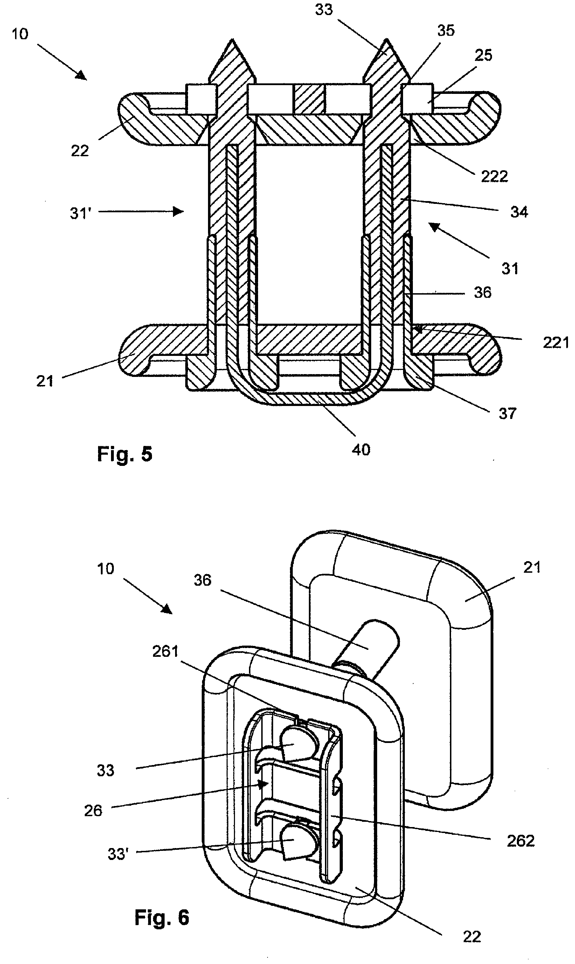

Wie insbesondere aus der Fig. 5 ersichtlich bestehen die Stifte 31, 31' aus einer Spitze 33 und einem Stiel 34, wobei zwischen Spitze 33 und Stiel 34 eine Einschnürung 35 vorgesehen ist, die im Wesentlichen durch eine Verringerung des Stiftdurchmessers erzielt ist. Der Stiel 34 des Stifts 31 ist in einen Schaft 36 eingesteckt und ist gleichzeitig mit dem Faden 40 unlösbar verbunden.

Der Schaft 36 ist durch eine in dem ersten Plattenelement 21 angeordneten Bohrung 221 hindurchgeführt und weist an seinem dem Stift 31 abgewandten Ende einen Kopf 37 auf, der einen größeren Außendurchmesser als der Durchmesser der Bohrung 221 aufweist, um ein Auseinanderziehen der beiden Plattenelemente 21, 22 zu verhindern.

Die Spitze 33 des Stifts 31 ist durch eine im Wesentlichen konische Bohrung 222 des zweiten Plattenelements 22 geführt, wobei die Einschnürung 35 aus der Bohrung 222 hinausragt. In diese Einschnürung 35 greift eine Rastleiste 25 (Fig. 2) ein, um den Stift 31 in seiner Position hinsichtlich des zweiten Plattenelements 22 zu fixieren. Durch die konische Form der Bohrung 222 lässt sich die Spitze 33 beim Verbinden der beiden Plattenelemente 21, 22 besonders einfach durch die Bohrung 222 hindurchführen.

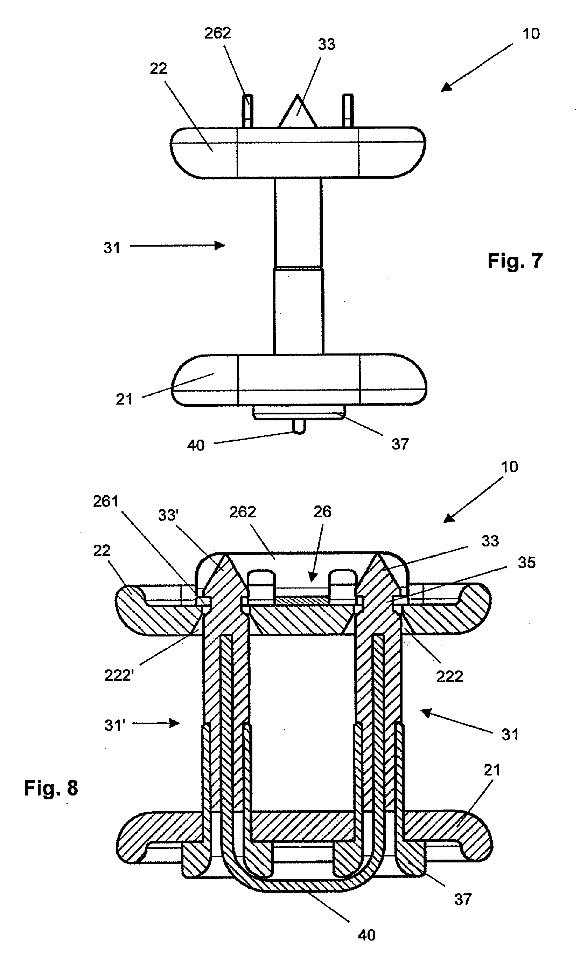

In einer alternativen Ausführung der Erfindung werden gemäß den Figuren 6 bis 8 die Stifte 31, 31' über ein Federelement 26 in ihrer Position hinsichtlich des zweiten Plattenelementes 22 fixiert, wobei das Federelement 26 elastisch ausgebildet ist. Beim Hindurchführen der Spitzen 33, 33' der Stifte 31, 31' durch die Bohrungen 222, 222', wobei die kegelförmige Spitze 33, 33' der Stifte 31, 31' das anschließende Durchdringen der vorgesehenen Öffnungen 261 des Federelementes 26 erleichtert, rastet das Federelement 26 in die Einschnürungen 35, die durch den reduzierten Querschnitt des Stiftes 31, 31' gebildet sind, ein und fixiert so die Stifte 31, 31'. Das Federelement 26 weist in dieser Ausführung erhöhte Flanken 262 auf, die das umliegende Gewebe vor Verletzung durch die Spitzen 33, 33' der Stifte 31, 31' schützen.

Wird nun der die beiden Stifte 31, 31' verbindende Faden 40 durchtrennt, wobei die erhöhte Ausformung des Schaftkopfes 37 dieses Durchtrennen wesentlich erleichtert, kann der aus dem ersten Plattenelement 21 sowie den beiden Schäften 36 bestehende Teil der erfindungsgemäßen Klammereinrichtung 10 auf einfache Weise von den Stiften 31, 31' abgezogen werden. Damit lässt sich die erfindungsgemäße Klammereinrichtung auf einfache Weise aus dem Gewebe lösen, ohne das umliegende Gewebe unnötig zu verletzen.

Es versteht sich, dass die Erfindung nicht auf das oben beschriebene Ausführungsbeispiel beschränkt ist. Insbesondere, können auch mehr als zwei, beispielsweise drei Stifte zur Verbindung zweier Plattenelemente vorgesehen sein. Wesentlich hierbei ist jedoch, dass jeder Stift mit zumindest einem weiteren Stift über einen Faden in Verbindung steht. Ebenso können auch andere Fixierungsmittel, insbesondere zur Fixierung der Stifte in dem zweiten Plattenelement vorgesehen sein.

## Patentansprüche

1. Chirurgische Klammereinrichtung (10) mit einem ersten und einem zweiten Plattenelement (21, 22), sowie zumindest zwei Stiften (31, 31'), über die das erste Plattenelement (21) mit dem zweiten Plattenelement (22) verbindbar ist, und die zumindest zwei Stifte (31, 31') an einem Ende über zumindest einen Faden (40) miteinander in Verbindung stehen, der die Stifte (31, 31) an dem ersten Plattenelement (21) fixiert, **dadurch gekennzeichnet, dass** je Stift (31, 31') ein Schaft (36, 36') vorgesehen ist, der an einem Ende den Stift (31, 31') trägt, während das andere Ende mit dem ersten Plattenelement (21) in Verbindung steht.

2. Klammereinrichtung (10) nach Anspruch 1, **dadurch gekennzeichnet, dass** die zumindest zwei Stifte (31, 31') über eine zusätzliche lösbare Verbindung an dem ersten Plattenelement (21) angeordnet sind, die bei Durchtrennung des zumindest einen Fadens (40) gelöst ist.

3. Klammereinrichtung (10) nach Anspruch 1 oder 2, **dadurch gekennzeichnet, dass** der Schaft (36, 36') in eine normal auf die Plattenebene stehende Bohrung (221) des ersten Plattenelementes (21) einsetzbar ist.

4. Klammereinrichtung (10) einem der Ansprüche 1 bis 3, **dadurch gekennzeichnet, dass** der Schaft (36, 36') an seinem dem ersten Plattenelement (21) zugewandten Ende einen Kopf (37) aufweist, dessen Durchmesser größer ist als der Durchmesser der Bohrung (221).

5. Klammereinrichtung (10) nach einem der Ansprüche 1 bis 4, **dadurch gekennzeichnet, dass** der Schaft (36, 36') einstückig mit dem ersten Plattenelement (21) gefertigt ist.

6. Klammereinrichtung (10) nach einem der Ansprüche 1 bis 5, **dadurch gekennzeichnet, dass** das zweite Plattenelement (22) zumindest zwei, normal auf die Plattenebene stehende Bohrungen (222) aufweist, in die die zumindest zwei Stifte (31, 31') einsetzbar sind.

7. Klammereinrichtung (10) nach einem der Ansprüche 1 bis 6, **dadurch gekennzeichnet, dass** das zweite Plattenelement (22) zumindest ein Fixierelement (25) aufweist, das zumindest einen Stift (31, 31') in seiner Position an dem zweiten Plattenelement (22) fixiert.

8. Klammereinrichtung (10) nach Anspruch 7, **dadurch gekennzeichnet, dass** der Stift (31, 31') eine im Wesentlichen kegelförmige Spitze (33) sowie in unmittelbarer Nachbarschaft der Spitze (33) einen reduzierten Querschnitt aufweist, wodurch eine Einschnürung (35) gebildet wird, in die das auf dem zweiten Plattenelement (22) angeordnete Fixierungsmittel (25, 26) einrastbar ist.

9. Klammereinrichtung (10) nach einem der Ansprüche 1 bis 8, **dadurch gekennzeichnet, dass** das Ende des zumindest einen Fadens (40) unlösbar mit dem Stift (31, 31') verbunden ist.

10. Klammereinrichtung (10) nach einem der Ansprüche 1 bis 9, **dadurch gekennzeichnet, dass** die Plattenelemente (21, 22) sowie die Stifte (31, 31') aus Titan oder Nitinol gefertigt sind.

11. Klammereinrichtung (10) nach einem der Ansprüche 1 bis 10, **dadurch gekennzeichnet, dass** der zumindest eine Faden (40) aus einem elastischen Material gefertigt ist.

## Claims

1. Surgical clamping device (10) with a first and a second plate element (21, 22) and two or more pins (31, 31') by means of which the first plate element (21) can be connected to the second plate element (22), said two or more pins (31, 31') being connected at one end via at least one thread (40) holding said pins (31, 31') on the first plate element (21), **characterised in that** for each pin (31, 31') a shaft (36, 36') is provided, which carries the pin (31, 31') at one end while the other end is connected to the first plate element (21).

2. Clamping device (10) according to claim 1, **characterised in that** said two or more pins (31, 31') are attached to the first plate element (21) by means of an additional releasable connection, which is released by cutting the thread (40).

3. Clamping device (10) according to claims 1 or 2, **characterised in that** the shaft (36, 36') can be inserted into a bore (221) of the first plate element (21), which bore (221) is perpendicular to the plane of the plate.

4. Clamping device (10) according to any of claims 1 to 3, **characterised in that** the shaft (36, 36') is provided with a head (37) at its end facing the first plate element (21), which head (37) has a diameter larger than the diameter of the bore (221).

5. Clamping device (10) according to any of claims 1 to 4, **characterised in that** the shaft (36, 36') and the first plate element (21) are manufactured as an integral unit.

6. Clamping device (10) according to any of claims 1 to 5, **characterised in that** the second plate element (22) has at least two bores (222) perpendicular to the plane of the plate, into which bores (222) the at least two pins (31, 31') can be inserted.

7. Clamping device (10) according to any of claims 1 to 6, **characterised in that** the second plate element (22) is provided with at least one locking element (25), which fixates at least one pin (31, 31') in its position on the second plate element (22).

8. Clamping device (10) according to claim 7, **characterised in that** the pin (31, 31') has an essentially conical tip (33) and that it has reduced cross-section in the immediate vicinity of the tip (33), such that a constriction (35) is formed in which the locking means (25, 26) positioned on the second plate element (22) can lock.

9. Clamping device (10) according to any of claims 1 to 8, **characterised in that** the end of the at least one thread (40) is permanently attached to the pin (31, 31').

10. Clamping device (10) according to any of claims 1 to 9, **characterised in that** the plate elements (21, 22) and the pins (31, 31') are made of titanium or nitinol.

11. Clamping device (10) according to any of claims 1 to 10, **characterised in that** the at least one thread (40) is made of an elastic material.

## Revendications

1. Dispositif de serrage chirurgical (10) comprenant un premier et un second élément de plaque (21, 22) ainsi qu'au moins deux broches (31, 31') permettant de relier le premier élément de plaque (21) et le second élément de plaque (22) et les deux broches (31, 31') étant reliées à l'une de leurs extrémités par au moins un fil (40) qui fixe les broches (31, 31') au premier élément de plaque (21),
**caractérisé en ce que**
pour chacune des broches (31, 31') il est prévu un arbre (36, 36') qui porte la broche (31, 31') à l'une de ses extrémités tandis que la seconde extrémité est reliée au premier élément de plaque (21).

2. Dispositif de serrage (10) conforme à la revendication 1,
**caractérisé en ce que**
les deux broches (31, 31') sont montées sur le premier élément de plaque (21) par l'intermédiaire d'une liaison amovible supplémentaire qui est supprimée suite au sectionnement du fil (40).

3. Dispositif de serrage (10) conforme à la revendication 1 ou 2,
**caractérisé en ce que**
l'arbre (36, 36') peut être inséré dans un perçage (221) du premier élément de plaque (21) disposé perpendiculairement au plan des plaques.

4. Dispositif de serrage (10) conforme à une des revendications 1 à 3,
**caractérisé en ce que** l'arbre (36, 36') comporte, à son extrémité tournée vers le premier élément de plaque (21) une tête (37) dont le diamètre est supérieur au diamètre du perçage (221).

5. Dispositif de serrage (10) conforme à une des revendications 1 à 4,
**caractérisé en ce que**
l'arbre (36, 36') est réalisé en une seule pièce avec le premier élément de plaque (21).

6. Dispositif de serrage (10) conforme à une des revendications 1 à 5,
**caractérisé en ce que**
le second élément de plaque (22) comporte au moins deux perçages (222) perpendiculaires au plan des plaques dans lesquels peuvent être introduites les deux broches (31, 31').

7. Dispositif de serrage (10) conforme à l'une des revendications 1 à 6,
**caractérisé en ce que**
le second élément de plaque (22) comporte au moins un élément de fixation (25) qui fixe au moins une broche (31, 31') dans sa position sur le second élément de plaque (22).

8. Dispositif de serrage (10) conforme à la revendication 7,
**caractérisé en ce que**
la broche (31, 31') comporte une pointe (33) essentiellement conique ainsi qu'à proximité immédiate de cette pointe (33) une section réduite de sorte que soit formée une gorge (35) dans laquelle peut être encliqueté le moyen de fixation (25, 26) monté sur le second élément de plaque (22).

9. Dispositif de serrage (10) conforme à l'une des revendications 1 à 8,
**caractérisé en ce que**
l'extrémité du fil (40) est reliée de façon indétachable à la broche (31,31').

10. Dispositif de serrage (10) conforme à l'une des revendications 1 à 9,
**caractérisé en ce que**
les éléments de plaque (21, 22) ainsi que les broches (31, 31') sont réalisés en titane ou en nitinol.

11. Dispositif de serrage (10) conforme à l'une des revendications 1 à 10,
**caractérisé en ce que**
le fil (40) est réalisé en un matériau élastique.
